# EUROPÄISCHE PATENTSCHRIFT

(11) **EP 0 518 061 B1**
(45) Veröffentlichungstag und Bekanntmachung des Hinweises auf die Patenterteilung: **06.12.1995**
(21) Anmeldenummer: 92107922.4
(22) Anmeldetag: 12.05.1992
(51) Int. Cl.: A61L 29/00, C08K 5/09, A61L 33/00

(54) **Medizinische Arbeitsmittel**
Medical articles
Articles médicaux

(30) Priorität: 23.05.1991 DE 4116812
(43) Veröffentlichungstag der Anmeldung: 16.12.1992
(73) Patentinhaber: REHAU AG + Co, 95111 Rehau (DE)
(72) Erfinder:

(56) Entgegenhaltungen:
- EP-A- 0 108 023
- CHEMICAL ABSTRACTS, vol. 77, no. 12, 18. September 1972, Columbus, Ohio, US; abstract no. 76126f, Seite 48; & JP-A-7 140 421

## Beschreibung

Die Erfindung betrifft medizinische Arbeitsmittel wie Katheter, Schläuche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen.

Aus der DE-A 19 30 136 ist ein beschichteter Katheter bekannt, welcher als Basis aus einem Schlauch aus Gummi oder einem Elastomerem besteht. Dieser Katheterschlauch ist an der Außen- und/oder Innenwand mit einem hydrophilen Polyacrylat bzw. -methacrylat, insbesondere mit einem Hydroxialcyl- oder einem Hydroxialcoxi-Alcyl-Acrylat bzw. -methacrylat mit jeweils niederen Acylresten beschichtet.

Weiterhin ist es bekannt, derartige medizinische Arbeitsmittel mit einer Beschichtung aus Heparin zu versehen. Diese Heparin-Beschichtungsverfahren arbeiten über APTES- und TDMAC-Brücken. Mit dieser Heparin-Beschichtung wird das Anhaften von Blutplättchen an der Oberfläche des medizinischen Arbeitsmittels verhindert und gleichzeitig die intrinsische Koagulation desaktiviert. Ausführungen zum Beschichtungsverfahren mit Heparin über TDMAC-Brücken finden sich bei G.A. Grode, R.D. Falb, J.P. Crowley in J.Biomed. Mater. Res. Symp., 3, 77 (1972). Entsprechende Hinweise zur Beschichtung mit Heparin über APTES-Brücken finden sich bei R.L. Merker, L.J. Elyash, S.W. Mayhew, J.Y.C. Wang in Proc. Artif. Heart Conf., 1969, Seite 29.

Alle diese Verfahren haben den prinzipiellen Nachteil, daß sie technisch aufwendig und sehr arbeitsintensiv sind. So läuft eine Heparinisierung bei beiden genannten Verfahren in mehreren zusätzlichen Arbeits- und Beschichtungszyklen ab.

Ein weiterer wesentlicher Nachteil dieser bekannten Verfahren ist, daß die auf diese Weise hergestellten medizinischen Halbzeuge sterilisiert werden müssen, weil die Beschichtung mit Heparin mikrobiell abgebaut werden kann. Da nach der Konfektion der Halbzeuge zu Fertigprodukten eine Gesamtsterilisation erforderlich ist, ist hier als weiterer Nachteil das erhebliche Risiko einer Mehrfachsterilisation zu nennen.

Hier setzt die Erfindung ein, die es sich zur Aufgabe gestellt hat, die zum Stand der Technik genannten Nachteile zu vermeiden und eine Methode zur Erzielung der optimalen Blutkompatibilität bei medizinischen Halbzeugen aufzuzeigen, bei der über das medizinische Arbeitsmittel weder das biologische System negativen Auswirkungen ausgesetzt ist, noch das Material, aus dem das medizinische Arbeitsmittel gefertigt ist, durch Einwirkung des biologischen Systems so weit geschädtigt werden kann, daß es die vorgesehene Funktion nicht mehr erfüllt. Erfindungsgemäß wird dazu vorgeschlagen, daß in das Ausgangspolymere vor der eigentlichen Formgebung eine definierte Menge von Seltenerdcarboxilaten als Zuschlagstoffe eingemischt sind.

Die Erfindung geht von der Überlegung aus, dem Ausgangswerkstoff Additive zuzusetzen, die den Werkstoff von der Blutkompatibilität her entweder in Richtung unendlich - Heparin - oder mit seinem relativen Gerinnungsparameter dem Quotienten 1,0 anzunähern. Dieses Vorhaben ist durch die Addition von Seltenerdcarboxilaten gelungen. So hat es sich bei der Verwendung von weichmacherhaltigem Polyvinylchlorid als Ausgangsmaterial für die Herstellung medizinischer Arbeitsmittel als vorteilhaft herausgestellt, daß die heute für solche Anwendungszwecke üblichen Stabilisatoren auf Basis Zink und Kalzium erfindungsgemäß durch Seltenerdcarboxilate ergänzt oder ersetzt werden. Hierbei hat sich als zweckmäßig eine Dosierungsrate von 0,02 bis 1,0 Gewichtsprozent erwiesen.

Bei der Verwendung aller anderen Polymere zur Herstellung der medizinischen Arbeitsmittel können die Seltenerdcarboxilate die eingesetzten Arbeitshilfen wie Gleitmittel und dergleichen ergänzen oder ersetzen. Hier haben sich Anteile zwischen 0,02 und 5 Gewichtsprozent als vorteilhaft erwiesen. Die erfindungsgemäßen Seltenerdcarboxilate werden in einer Partikelgröße von maximal 5» verwendet.

Die erfindungsgemäß mit dem Seltenerdcarboxilat-Zuschlag versehenen medizinischen Arbeitsmittel verhindern die Blutplättchen-Aggregation und desaktivieren die intrinsische Koagulation. Eine Vorsterilisierung der Halbzeuge ist nicht mehr erforderlich, da die erfindungsgemäßen Seltenerdcarboxilate entgegen der bisher üblichen Heparin-Beschichtung nicht mehr mikrobiell abgebaut werden können.

Die nachfolgenden Beispiele zeigen die Verwendungsmöglichkeit der erfindungsgemäßen Seltenerdcarboxilate als Zuschlagstoffe für polymere Materialien zur Herstellung medizinischer Arbeitsmittel.

### Beispiel 1:

### Weich-PVC-Rezeptur

100 Teile S-PVC
50 Teile Weichmacher
5 Teile epoxidiertes Sojaöl
0,1 Teil Kalziumstearat
0,1 Teil Zinkstearat
0,1 Teil Neodymstearat

### Beispiel 2:

### Weich-PVC-Rezeptur

100 Teile S-PVC
50 Teile Weichmacher
5 Teile epoxidiertes Sojaöl
0,12 Teile Kalziumstearat
0,15 Teile Lanthanstearat

### Beispiel 3:

### Weich-PVC-Rezeptur

100 Teile S-PVC
50 Teile Weichmacher
5 Teile epoxidiertes Sojaöl
0,15 Teile Zinkstearat
0,15 Teile Kalziumstearat
0,5 Teile Samariumstearat

### Beispiel 4:

### Weich-PVC-Rezeptur

100 Teile S-PVC
16 Teile Weichmacher
5 Teile epoxidiertes Sojaöl
0,2 Teile Ceroctoat
0,15 Teile Dysprosiumstearat

### Beispiel 5:

### Weich-PVC-Rezeptur

100 Teile S-PVC
50 Teile Weichmacher
8 Teile epoxidiertes Sojaöl
0,2 Teile Samariumstearat
0,3 Teile Cerstearat
Beim Beispiel 5 ersetzen die Seltenerdcarboxilate gänzlich die üblichen Stabilisatoren.

### Beispiel 6:

### Polyurethan-Rezeptur

100 Teile Polyurethan
3 Teile Neodymstearat
Bei diesem Beispiel ersetzen die Seltenerdcarboxilate die herkömmlichen Gleitmittel.

### Beispiel 7:

### Polyurethan-Rezeptur

100 Teile Polyurethan
1,5 Teile Neodymstearat
1,5 Teile N, N-Diacyl-Ethylen-Diamin

### Beispiel 8:

### Polyethylen-Rezeptur

100 Teile Polyethylen
5 Teile Cerstearat
Auch bei diesem Beispiel ersetzen die Seltenerdcarboxilate die herkömmlichen Gleitmittel.

### Beispiel 9:

### Polypropylen-Rezeptur

100 Teile Polypropylen
3 Teile Cerstearat
2 Teile N, N-Diacyl-Ethylen-Diamin
Bei diesem Beispiel ergänzen die Seltenerdcarboxilate die herkömmlichen Gleitmittel.

## Patentansprüche

1. Medizinische Arbeitsmittel wie Katheter, Schläuche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen, dadurch gekennzeichnet, daß in das Ausgangspolymere vor der eigentlichen Formgebung eine definierte Menge von Seltenerdcarboxilaten als Zuschlagstoffe eingemischt sind.

2. Arbeitsmittel nach Anspruch 1, dadurch gekennzeichnet, daß die Menge der Seltenerdcarboxilate zwischen 0,02 und 5 Gewichtsprozent liegt.

3. Arbeitsmittel nach Ansprüchen 1 und 2, dadurch gekennzeichnet, daß die Seltenerdcarboxilate als Ergänzung und/oder Ersatz der weiteren Zuschlagstoffe des Ausgangspolymeren wie Stabilisatoren, Gleitmittel und dergleichen dienen.

4. Arbeitsmittel nach Anspruch 1, dadurch gekennzeichnet, daß die Seltenerdcarboxilate in einer Partikelgröße von maximal 5» verwendet werden.

## Claims

1. Medical products such as catheters, tubings, containers, mouldings and similar articles made of polymer material, whereby these medical products are specially designed to increase blood compatibility when in contact with blood or blood-like liquids, characterized in that, before shaping, the base polymer is mixed with a certain amount of rare-earth carboxilates as additives.

2. Products as described in point 1, characterized in that the amount of rare-earth carboxilates is between 0.02 and 5 percent of the weight.

3. Products as described in points 1 and 2, characterized in that the rare-earth carboxilates serve as additions and/or replacements to other additives such as stabilizers, lubricants and similar substances.

4. Products as described in point 1 characterized in that the maximum particle size of the rare-earth carboxilates is 5 ».

## Revendications

1. Articles de médecine tels que cathéters, tubulures, réservoirs, pièces moulées et autres articles similaires en matières synthétiques, mis au point spécialement de sorte à augmenter la compatibilité sanguine en cas de contact avec le sang ou liquides similaires, caractérisé en ce qu'une quantité définie de carboxylates des terres rares est incorporée dans la matière de base avant sa mise en forme.

2. Articles selon la revendication 1, caractérisés en ce que la quantité de carboxylates des terres rares incorporée se situe entre 0,02 et 5 % en masse.

3. Articles selon les revendications 1 et 2, caractérisés en ce que les carboxylates des terres rares servent à compléter et/ou à remplacer les autres adjuvants de la matière de base, tels que stabilisants, lubrifiants et autres substances similaires.

4. Articles selon la revendication 1, caractérisés en ce que les particules des carboxylates des terres rares ont une dimension maximale de 5 ».
